# EUROPEAN PATENT APPLICATION

(11) **EP 4 501 897 A1**
(43) Date of publication of application: **05.02.2025**
(21) Application number: 23779740.2
(22) Date of filing: 17.03.2023
(51) Int. Cl.: C07C 45/27, C07C 45/56, C07C 45/66, C07C 49/385, C07C 49/587, C07B 61/00, B01J 23/28, B01J 27/128, B01J 31/22

(54) **METHOD FOR PRODUCING CYCLIC DIKETONE COMPOUND**

(30) Priority: 31.03.2022 JP 2022060671
(71) Applicant: Kao Corporation, Tokyo 103-8210 (JP)
(72) Inventor: KASHIWAGI, Yuki, Tokyo 131-8501 (JP); DEGUCHI, Jun, Tokyo 131-8501 (JP)
(74) Representative: Hoffmann Eitle
(86) International application number: PCT/JP2023/010574
(87) International publication number: WO 2023/189723

(57) **Abstract**

Provided is a method for producing a compound represented by General Formula (I) through oxidative cleavage of a compound of Formula (II), which is a bicyclic tetrasubstituted olefin compound. This method is for producing a compound represented by General Formula (I), including a step of obtaining a compound represented by General Formula (I) through oxidative cleavage of a compound represented by General Formula (II) using an oxidant in the presence of a metal catalyst containing one or more metal elements selected from the group consisting of vanadium, iron, and molybdenum. 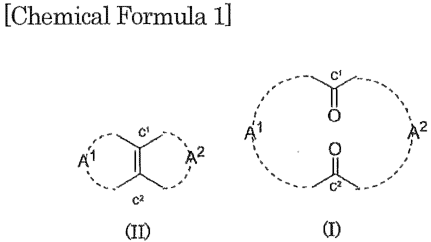 In Formulas (I) and (II) above, Formula -A¹- (wherein a former bonding hand means a bonding hand that is bonded to a carbon atom C¹, and a latter bonding hand means a bonding hand that is bonded to a carbon atom C²) is an alkylene group with 2 or more and 6 or less of carbon atoms that is optionally substituted and that optionally further contains an ether bond, an ester bond, a secondary amino group, a thioether group, or a combination thereof, and Formula -A²- (wherein a former bonding hand means a bonding hand that is bonded to a carbon atom C¹, and a latter bonding hand means a bonding hand that is bonded to a carbon atom C²) is an alkylene group with 4 or more and 10 or less of carbon atoms that is optionally substituted and that optionally further contains an ether bond, an ester bond, a secondary amino group, a thioether group, or a combination thereof.

## Description

### Technical Field

This invention relates to a method for producing a compound represented by General Formula (I).

### Background Art

Macrocyclic compounds are known to exhibit useful activity in pharmaceuticals, fragrances, and agrochemicals. Muscenone, which is a type of macrocyclic ketones, is a fragrance material with excellent biodegradability, high residual fragrance, and elegant texture. Various production methods have been disclosed to meet the growing needs for readily degradable synthetic musk materials in recent years.

JP S51-24498B (Patent Document 1) discloses the production of cyclopentadecane-1,5-dione through oxidative cleavage of a tetrasubstituted double bond in 14-methylbicyclo[10.3.0]pentadecene[1(12)] with ozone or potassium permanganate.

JP 2016-124867A (Patent Document 2) discloses a method for producing a diketone compound through oxidative cleavage using hydrogen peroxide in the presence of an acid catalyst or a tungstic acid compound.

### Disclosure of Invention

The present invention is directed to a method for producing a compound represented by General Formula (I), including a step of obtaining a compound represented by General Formula (I) through oxidative cleavage of a compound represented by General Formula (II) using an oxidant in the presence of a metal catalyst containing one or more metal elements selected from the group consisting of vanadium, iron, and molybdenum.

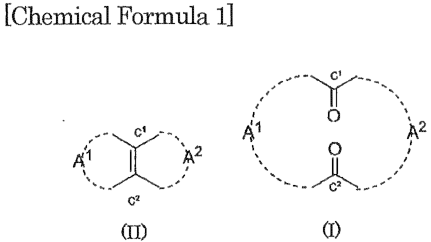

In Formulas (I) and (II) above,
Formula -A¹- (wherein a former bonding hand means a bonding hand that is bonded to a carbon atom C¹, and a latter bonding hand means a bonding hand that is bonded to a carbon atom C²) is an alkylene group with 2 or more and 6 or less of carbon atoms that is optionally substituted and that optionally further contains an ether bond, an ester bond, a secondary amino group, a thioether group, or a combination thereof, and
Formula -A²- (wherein a former bonding hand means a bonding hand that is bonded to a carbon atom C¹, and a latter bonding hand means a bonding hand that is bonded to a carbon atom C²) is an alkylene group with 4 or more and 10 or less of carbon atoms that is optionally substituted and that optionally further contains an ether bond, an ester bond, a secondary amino group, a thioether group, or a combination thereof.

### Description of the Invention

The versatility of the method of Patent Document 1 is low because of the use of ozone, and thus this method can hardly be said to be industrially suitable.

It is an object of the present invention to provide a method for producing a compound represented by General Formula (I) through oxidative cleavage of a compound of Formula (II), which is a bicyclic tetrasubstituted olefin compound.

In order to solve the above-described problems, the inventors of the present invention conducted an in-depth study on the oxidative cleavage of tetrasubstituted olefin compounds using oxidants, and surprisingly found that, in the presence of a specific metal catalyst, the oxidative cleavage reaction of a bicyclic tetrasubstituted olefin compound proceeds.

That is to say, the present invention is directed to a method for producing a compound represented by General Formula (I), including a step of obtaining a compound represented by General Formula (I) through oxidative cleavage of a compound represented by General Formula (II) using an oxidant in the presence of a metal catalyst containing one or more metal elements selected from the group consisting of vanadium, iron, and molybdenum.

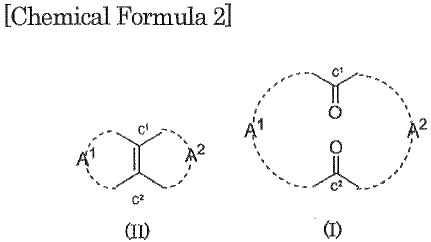

In Formulas (I) and (II) above,
Formula -A¹- (wherein the former bonding hand means a bonding hand that is bonded to a carbon atom C¹, and the latter bonding hand means a bonding hand that is bonded to a carbon atom C²) is an alkylene group with 2 or more and 6 or less of carbon atoms that is optionally substituted and that optionally further contains an ether bond, an ester bond, a secondary amino group, a thioether group, or a combination thereof, and
Formula -A²- (wherein the former bonding hand means a bonding hand that is bonded to a carbon atom C¹, and the latter bonding hand means a bonding hand that is bonded to a carbon atom C²) is an alkylene group with 4 or more and 10 or less of carbon atoms that is optionally substituted and that optionally further contains an ether bond, an ester bond, a secondary amino group, a thioether group, or a combination thereof.

According to the present invention, it is possible to produce a compound represented by General Formula (I) through oxidative cleavage of a compound of Formula (II), which is a bicyclic tetrasubstituted olefin compound.

The present invention is directed to a method for producing a compound represented by General Formula (I), including a step of obtaining a compound represented by General Formula (I) (hereinafter, also referred to as a "compound of Formula (I)") through oxidative cleavage of a compound represented by General Formula (II) (hereinafter, also referred to as a "compound of Formula (II)") using an oxidant in the presence of a metal catalyst containing one or more metal elements selected from the group consisting of vanadium, iron, and molybdenum.

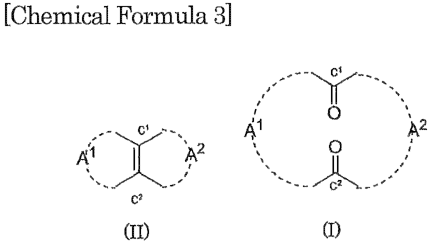

In Formulas (I) and (II) above,
Formula -A¹- (wherein the former bonding hand means a bonding hand that is bonded to a carbon atom C¹, and the latter bonding hand means a bonding hand that is bonded to a carbon atom C²) is an alkylene group with 2 or more and 6 or less of carbon atoms that is optionally substituted and that optionally further contains an ether bond, an ester bond, a secondary amino group, a thioether group, or a combination thereof, and
Formula -A²- (wherein the former bonding hand means a bonding hand that is bonded to a carbon atom C¹, and the latter bonding hand means a bonding hand that is bonded to a carbon atom C²) is an alkylene group with 4 or more and 10 or less of carbon atoms that is optionally substituted and that optionally further contains an ether bond, an ester bond, a secondary amino group, a thioether group, or a combination thereof.

Although the reasons why the compound represented by General Formula (I) can be produced through oxidative cleavage of a bicyclic tetrasubstituted olefin compound using an oxidant in the presence of a metal catalyst are not clear, it is presumed that two ring structures constrain the degree of freedom of the four bonds that are bonded to the double bond, which makes the oxidative cleavage possible.

Compound of Formula (I) and Compound of Formula (II)

In Formulas (II) and (I) above, the "alkylene group with 2 or more and 6 or less of carbon atoms" of the "alkylene group with 2 or more and 6 or less of carbon atoms that is optionally substituted and that optionally further contains an ether bond, an ester bond, a secondary amino group, a thioether group, or a combination thereof" of Formula -A¹- is represented by, for example, Formula -(CH₂)₂-, Formula - (CH₂)₃-, Formula -(CH₂)₄-, Formula -(CH₂)₅-, or Formula -(CH₂)₆-, and is preferably Formula -(CH₂)₃- or Formula -(CH₂)₄-. The alkylene group with 2 or more and 6 or less of carbon atoms that is optionally substituted is preferably an alkylene group with 3 or 4 carbon atoms that is optionally substituted.

In Formulas (II) and (I) above, the "alkylene group with 2 or more and 6 or less of carbon atoms that optionally further contains an ether bond, an ester bond, a secondary amino group, a thioether group, or a combination thereof' of the "alkylene group with 2 or more and 6 or less of carbon atoms that is optionally substituted and that optionally further contains an ether bond, an ester bond, a secondary amino group, a thioether group, or a combination thereof" of Formula -A¹- is, for example, an alkylene group with 2 or more and 6 or less of carbon atoms that contains one or a combination of two or more selected from the group consisting of an ether bond (-O-), an ester bond (-C(=O)-O- or -O-C(=O)-), a secondary amino group (-NH-), and a thioether group (-S-). Note that the 2 or more and 6 or less of carbon atoms do not include any carbon atoms of an ester bond. The "alkylene group with 2 or more and 6 or less of carbon atoms that optionally further contains an ether bond, an ester bond, a secondary amino group, a thioether group, or a combination thereof' may be, for example, Formula -CH₂-O-CH₂-, Formula -CH₂-C(=O)-OCH₂-, Formula -CH₂-NH-CH₂-, Formula -CH₂-S-CH₂-, Formula -(CH₂)₂-O-CH₂-, Formula -(CH₂)₂-NH-(CH₂)₂-, Formula -(CH₂)₂-, Formula -(CH₂)₃-, Formula -(CH₂)₄-, Formula -(CH₂)₅-, Formula - (CH₂)₆-, or the like, and preferably Formula -(CH₂)₃- or Formula -(CH₂)₄-.

In Formulas (II) and (I) above, the "alkylene group with 4 or more and 10 or less of carbon atoms" of the "alkylene group with 4 or more and 10 or less of carbon atoms that is optionally substituted and that optionally further contains an ether bond, an ester bond, a secondary amino group, a thioether group, or a combination thereof" of Formula -A²- is represented by, for example, Formula -(CH₂)₄-, Formula - (CH₂)₅-, Formula -(CH₂)₆-, Formula -(CH₂)₇-, Formula -(CH₂)₈-, Formula -(CH₂)₉-, or Formula -(CH₂)₁₀-, preferably Formula -(CH₂)₄-, Formula -(CH₂)₆-, Formula -(CH₂)₈-, or Formula -(CH₂)₁₀-, and more preferably Formula -(CH₂)₄-, Formula -(CH₂)₆-, or Formula -(CH₂)₁₀-. The alkylene group with 4 or more and 10 or less of carbon atoms that is optionally substituted is preferably an alkylene group with 4, 6, 8, or 10 carbon atoms that is optionally substituted, and more preferably an alkylene group with 4, 6, or 10 carbon atoms.

In Formulas (II) and (I) above, the "alkylene group with 4 or more and 10 or less of carbon atoms that optionally further contains an ether bond, an ester bond, a secondary amino group, a thioether group, or a combination thereof' of the "alkylene group with 4 or more and 10 or less of carbon atoms that is optionally substituted and that optionally further contains an ether bond, an ester bond, a secondary amino group, a thioether group, or a combination thereof' of Formula -A²- is, for example, an alkylene group with 4 or more and 10 or less of carbon atoms that contains one or a combination of two or more selected from the group consisting of an ether bond (-O-), an ester bond (-C(=O)-O- or -O-C(=O)-), a secondary amino group (-NH-), and a thioether group (-S-). Note that the 4 or more and 10 or less of carbon atoms do not include any carbon atoms of an ester bond. The "alkylene group with 4 or more and 10 or less of carbon atoms that optionally further contains an ether bond, an ester bond, a secondary amino group, a thioether group, or a combination thereof' is, for example, Formula -(CHO₂-O-(CH₂)₂-, Formula -(CH₂)₂-NH-(CH₂)₂-, Formula -(CH₂)₄-, Formula -(CH₂)₅-, Formula -(CH₂)₆-, Formula -(CH₂)₇-, Formula -(CH₂)₈-, Formula - (CH₂)₉-, Formula -(CH₂)₁₀-, or the like, preferably Formula -(CH₂)₂-O-(CH₂)₂-, Formula -(CH₂)₄-, Formula -(CH₂)₆-, Formula -(CH₂)₈-, or Formula -(CH₂)₁₀-, and more preferably Formula -(CH₂)₄-, Formula -(CH₂)₆-, or Formula -(CH₂)₁₀-.

The "alkylene group with 2 or more and 6 or less of carbon atoms that is optionally substituted and that optionally further contains an ether bond, an ester bond, a secondary amino group, a thioether group, or a combination thereof" of Formula -A¹- and the "alkylene group with 4 or more and 10 or less of carbon atoms that is optionally substituted and that optionally further contains an ether bond, an ester bond, a secondary amino group, a thioether group, or a combination thereof" of Formula -A²- are respectively an "alkylene group with 2 or more and 6 or less of carbon atoms that optionally further contains an ether bond, an ester bond, a secondary amino group, a thioether group, or a combination thereof' and an "alkylene group with 4 or more and 10 or less of carbon atoms that optionally further contains an ether bond, an ester bond, a secondary amino group, a thioether group, or a combination thereof", the alkylene groups optionally having one or more substituents, and preferably one or two substituents. The substituents may be an alkyl group, an alkoxy group, an alkylamino group, an alkoxycarbonyl group, an alkanoyl group, an aryl group, an aralkyl group, an aryloxy group, an acyloxy group, a carboxy group, a halogen atom, a carbocyclic ring, a heterocyclic ring, or the like, and are preferably an alkyl group, an alkoxycarbonyl group, or an alkoxy group, and more preferably an alkyl group.

The alkyl group may be, for example, an alkyl group with 1 or more and 6 or less of carbon atoms, and is preferably an alkyl group with 1 or more and 4 or less of carbon atoms, and more preferably an alkyl group with 1 or 2 carbon atoms.

The alkoxy group may be, for example, an alkoxy group with 1 or more and 6 or less of carbon atoms, and is preferably an alkoxy group with 1 or more and 4 or less of carbon atoms, and more preferably an alkoxy group with 1 or 2 carbon atoms.

The alkylamino group may be, for example, an alkylamino group with 1 or more and 6 or less of carbon atoms, and is preferably an alkylamino group with 1 or more and 4 or less of carbon atoms, and more preferably an alkylamino group with 1 or 2 carbon atoms. The alkylamino group may be either a monoalkylamino group or a dialkylamino group.

The alkoxycarbonyl group may be, for example, an alkoxycarbonyl group with 1 or more and 6 or less of carbon atoms in the alkyl moiety, and is preferably an alkoxycarbonyl group with 1 or more and 4 or less of carbon atoms in the alkyl moiety, and more preferably an alkoxycarbonyl group with 1 or 2 carbon atoms in the alkyl moiety.

The alkanoyl group may be, for example, an alkanoyl group with 1 or more and 6 or less of carbon atoms in the alkyl moiety, and is preferably an alkanoyl group with 1 or more and 4 or less of carbon atoms in the alkyl moiety, and more preferably an alkanoyl group with 1 or 2 carbon atoms in the alkyl moiety.

The aryl group may be, for example, an aryl group with 6 or more and 10 or less of carbon atoms.

The aralkyl group means an arylalkyl group, but may be, for example, an aralkyl group with 6 or more and 10 or less of carbon atoms in the aryl moiety and 1 or more and 6 or less of carbon atoms in the alkyl moiety, and is preferably an aralkyl group with 6 or more and 10 or less of carbon atoms in the aryl moiety and 1 or more and 4 or less of carbon atoms in the alkyl moiety, and more preferably an aralkyl group with 6 or more and 10 or less of carbon atoms in the aryl moiety and 1 or 2 carbon atoms in the alkyl moiety.

The aryloxy group may be, for example, an aryloxy group with 6 or more and 10 or less of carbon atoms.

The acyloxy group may be, for example, an alkylcarbonyloxy group with 1 or more and 6 or less of carbon atoms in the alkyl moiety, and is preferably an alkylcarbonyloxy group with 1 or more and 4 or less of carbon atoms in the alkyl moiety, and more preferably an alkylcarbonyloxy group with 1 or 2 carbon atoms in the alkyl moiety.

The halogen atom may be fluorine, chlorine, bromine, or iodine.

The carbocyclic ring means a saturated or unsaturated cyclic hydrocarbon, and may be, for example, a saturated cyclic hydrocarbon with 3 or more and 10 or less of carbon atoms or an unsaturated cyclic hydrocarbon with 4 or more and 10 or less of carbon atoms.

The heterocyclic ring means a saturated or unsaturated cyclic hydrocarbon containing a heteroatom (e.g., oxygen, nitrogen, sulfur, etc.), and may be, for example, a saturated cyclic hydrocarbon with 3 or more and 10 or less of carbon atoms containing a heteroatom or an unsaturated cyclic hydrocarbon with 4 or more and 10 or less of carbon atoms containing a heteroatom.

Two or more of the above-mentioned substituents may be bonded to each other to form a carbocyclic ring or a heterocyclic ring.

The "alkylene group with 2 or more and 6 or less of carbon atoms that is optionally substituted and that optionally further contains an ether bond, an ester bond, a secondary amino group, a thioether group, or a combination thereof' of Formula -A¹- may be, for example, Formula -CH₂-O-CH₂-, Formula -CH₂-C(=O)-O-CH₂-, Formula -CH₂-NH-CH₂-, Formula -CH₂-S-CH₂-, Formula -(CH₂)₂-O-CH₂-, Formula -(CH₂)₂-NH-(CH₂)₂-, Formula -(CH₂)₂-, Formula -(CH₂)₃-, Formula -(CH₂)₄-, Formula -(CH₂)₅-, Formula -(CH₂)₆-, or the like, which is optionally substituted, and is preferably Formula -(CH₂)₃- or Formula -(CH₂)₄-, which is optionally substituted, and more preferably Formula -(CH₂)₈-, Formula -(CH₂)₄-, or Formula -CH₂-CH(CH₃)-CH₂-.

The "alkylene group with 4 or more and 10 or less of carbon atoms that is optionally substituted and that optionally further contains an ether bond, an ester bond, a secondary amino group, a thioether group, or a combination thereof' of Formula -A²- may be, for example, Formula -(CHO₂-O-(CH₂)₂-, Formula -(CH₂)₂-NH-(CH₂)₂-, Formula -(CH₂)₄-, Formula -(CH₂)₅-, Formula -(CH₂)₆-, Formula -(CH₂)₇-, Formula -(CHz)s-, Formula -(CH₂)₉-, Formula -(CH₂)₁₀-, or the like, which is optionally substituted, and is preferably Formula -(CHO₂-O-(CH₂)₂-, Formula -(CH₂)₄-, Formula - (CH₂)₆-, Formula -(CH₂)₈-, or Formula -(CH₂)₁₀-, which is optionally substituted, more preferably Formula -(CH₂)₂-O-(CH₂)₂-, Formula -(CH₂)₄-, Formula -(CH₂)₆-, Formula - (CH₂)₈-, or Formula -(CH₂)₁₀-, and even more preferably Formula -(CH₂)₄-, Formula - (CH₂)₆-, or Formula -(CH₂)₁₀-.

From the viewpoint of using the obtained compound of General Formula (I) as a precursor of a fragrance compound, Formula -A¹- above (wherein the former bonding hand means a bonding hand that is bonded to a carbon atom C¹, and the latter bonding hand means a bonding hand that is bonded to a carbon atom C²) is preferably an alkylene group with 3 or 4 carbon atoms that is optionally substituted, more preferably Formula -(CH₂)₃-, Formula -CH₂-CH(CH₃)-CH₂-, or Formula -(CH₂)₄-, and even more preferably Formula -CH₂-CH(CH₃)-CH₂-.

From the viewpoint of using the obtained compound of General Formula (I) as a precursor of a fragrance compound, Formula -A²- above (wherein the former bonding hand means a bonding hand that is bonded to a carbon atom C¹, and the latter bonding hand means a bonding hand that is bonded to a carbon atom C²) is preferably an alkylene group with 4, 6, 8 or 10 carbon atoms that is optionally substituted, more preferably Formula -(CH₂)₄-, Formula -CH₂-CH(CH₃)-CH₂-CH₂-, Formula -CH₂-CH₂-CH (CH₃)-CH₂-, Formula -(CH₂)₆-, Formula -(CH₂)₈-, or Formula -(CH₂)₁₀-, even more preferably Formula -(CH₂)₄-, Formula -(CH₂)₆-, Formula -(CH₂)₈-, or Formula -(CH₂)₁₀-, even more preferably Formula -(CH₂)₄-, Formula -(CH₂)₆-, or Formula -(CH₂)₁₀-, and even more preferably Formula -(CH₂)₁₀-.

The compound represented by General Formula (II) above is, for example, represented by the following formula, and, from the viewpoint of using the obtained compound of General Formula (I) as a precursor of a fragrance compound, the compound is preferably a compound represented by Formula (i), a compound represented by Formula (iii), a compound represented by Formula (vii), or a compound represented by Formula (viii), and more preferably a compound represented by Formula (vii). The compound represented by Formula (vii) is 14-methylbicyclo [10.3. O]pentadecene [1 (12)].

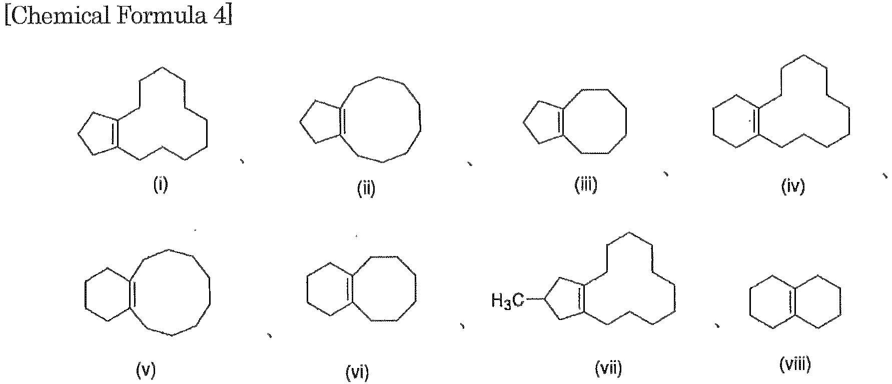

The compound represented by General Formula (I) above is, for example, 3-methyl-1,5-cyclopentadecanedione represented by Formula (1) below.

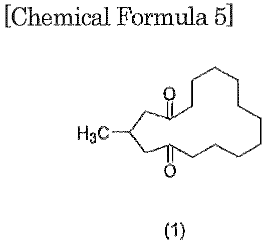

### Metal Catalyst

The oxidative cleavage is performed in the presence of a metal catalyst containing one or more metal elements selected from the group consisting of vanadium, iron, and molybdenum. The vanadium is preferably one or more selected from a vanadic acid compound, a vanadate, a vanadium alkoxide compound, and a vanadium complex compound. The molybdenum is preferably one or more selected from a molybdic acid compound and a molybdenum complex compound. The iron is preferably one or more selected from iron oxide, iron nitrate, iron sulfate, iron alkoxide, iron carboxylate, iron halide, and iron complex. Examples of the metal catalyst include a vanadium(IV) compound, a vanadium(V) compound, a molybdenum(VI) compound, an iron(II) compound, and an iron(III) compound, which may be used alone or in a combination of two or more. The metal catalyst is preferably molybdenum(VI) oxide, molybdic acid, dioxomolybdenum(VI) complex, polymolybdic acid, or heteropolymolybdic acid.

The vanadium(IV) compound and the vanadium(V) compound may be vanadium(V) oxide, vanadate(V), vanadium(V) oxytrialkoxide, vanadium(IV) complex, or the like. The vanadate(V) may be ammonium orthovanadate(V), ammonium metavanadate(V), sodium orthovanadate(V), sodium metavanadate(V), or the like. The vanadium oxytrialkoxide may be vanadium(V) oxytrimethoxide, vanadium(V) oxytriethoxide, vanadium(V) oxytriisopropoxide, vanadium(V) oxytri-tert-butoxide, vanadium(V) oxytri-sec-butoxide, or the like. Examples of the vanadium(IV) complex include vanadium(IV) oxide acetylacetonato complex VO(acac)₂, vanadium(IV) oxide phthalocyanine complex, vanadium(IV) oxide porphyrin complex, and vanadium(IV) oxide salen complex.

The molybdenum(VI) compound may be molybdenum(VI) oxide (MoO₃), molybdic acid (H₂MoO₄), polymolybdic acid, heteropolymolybdic acid, dioxomolybdenum(VI) complex, or the like. Examples of the dioxomolybdenum(VI) complex include dioxomolybdenum(VI) acetylacetonato complex MoO₂(acac)₂, dioxomolybdenum(VI) porphyrin complex, and dioxomolybdenum(VI) salen complex. The polymolybdic acid has a structure in which molybdic acid is condensed, and examples thereof include metamolybdic acid (H₆[H₂Mo₁₂O₄₀]) and paramolybdic acid (H₆[H₁₀Mo₁₂O₄₆]). The heteropolymolybdic acid has a structure in which molybdic acid is condensed with a heteroatom such as a phosphorus or silicon atom, and examples thereof include phosphomolybdic acid (H₃PMo₁₂O₄₀(nH₂O)), silimolybdic acid (H₄[SiMo₁₂O₄₀](nH₂O)), and phosphovanadomolybdic acid (H_{15-X}[PV₁₂-_{X}MoₓO₄₀]·(nH₂O)).

The iron(II) compound and the iron(III) compound may be iron(II) oxide, iron(III) oxide, iron(II) nitrate, iron(III) nitrate, iron(II) sulfate, iron(III) sulfate, iron alkoxide, iron carboxylate, iron halide, iron complex, or the like. The iron alkoxide may be iron(II) methoxide, iron(III) methoxide, iron(II) ethoxide, iron(III) ethoxide, iron(II) isopropoxide, iron(III) isopropoxide, iron(II) tert-butoxide, iron(III) tert-butoxide, iron(II) sec-butoxide, iron (III) sec-butoxide, or the like. The iron carboxylate may be iron(II) formate, iron(III) formate, iron(II) acetate, iron(III) acetate, iron(II) trifluoroacetate, iron (III) trifluoroacetate, or the like. The iron halide may be iron(II) chloride, iron(III) chloride, iron(II) bromide, iron (III) bromide, or the like. The iron complex may be iron(II) acetylacetonato complex, iron(III) acetylacetonato complex, iron porphyrin complex, iron salen complex, or the like.

The molar ratio of the metal catalyst to the compound of Formula (II) [metal catalyst / compound of Formula (II)] is, for example, 0.0001 or more, preferably 0.001 or more, and more preferably 0.01 or more, from the viewpoint of improving the reaction yield, and is, for example, 1 or less, preferably 0.7 or less, and more preferably 0.3 or less, from the viewpoint of reducing the production cost. The molar ratio of the metal catalyst to the compound of Formula (II) [metal catalyst / compound of Formula (II)] is, for example, 0.0001 or more and 1 or less, preferably 0.001 or more and 0.7 or less, and more preferably 0.01 or more and 0.3 or less, from the viewpoint of improving the reaction yield.

### Oxidant

Examples of the oxidant include peroxides, halogen acids and salts thereof, and perhalogen acids and salts thereof. These oxidants may be used alone or in a combination of two or more. Examples of the peroxides include peracids and salts thereof, non-peracid type organic peroxides, and non-peracid type inorganic peroxides. Examples of the peracids include percarboxylic acid, persulfuric acid, percarbonic acid, perphosphoric acid, and hypo-perhalogen acid. Examples of the non-peracid type organic peroxides include tert-butyl hydroperoxide, cumene hydroperoxide, di-tert-butyl peroxide, dimethyldioxirane, acetone peroxide, methyl ethyl ketone peroxide, and hexamethylenetriperoxidediamine. Examples of the non-peracid type inorganic peroxides include hydrogen peroxide, lithium peroxide, sodium peroxide, potassium peroxide, and permanganate. Examples of the halogen acids include chloric acid, bromic acid, and iodic acid. Examples of the perhalogen acids include perchloric acid, perbromic acid, and periodic acid. The oxidant is not limited, but is preferably hydrogen peroxide or tert-butyl hydroperoxide (TBHP) from the viewpoint of production cost and ease of handling.

The molar ratio of the oxidant to the compound represented by General Formula (II) [oxidant / compound represented by General Formula (II)] is, for example, 0.5 or more, preferably 1 or more, and more preferably 1.2 or more, from the viewpoint of yield, and is, for example, 20 or less, preferably 10 or less, and more preferably 5 or less, from the viewpoint of reducing the production cost. The molar ratio of the oxidant to the compound represented by General Formula (II) [oxidant / compound represented by General Formula (II)] is, for example, 0.5 or more and 20 or less, preferably 1 or more and 10 or less, and more preferably 1.2 or more and 5 or less, from the viewpoint of yield.

In the case of using hydrogen peroxide as the oxidant, the concentration of the hydrogen peroxide is not particularly limited, but is, for example, 1 to 80 mass%, and may be selected from the range of preferably 30 to 60 mass% from the viewpoint of safety and reaction yield.

### Additive

In the present invention, the oxidative cleavage may be performed further in the presence of an additive. Examples of the additive include inorganic acids such as phosphoric acid, sulfuric acid, and hydrochloric acid; polycarboxylic acids such as oxalic acid; organic acids such as p-toluenesulfonic acid; and a compound represented by Formula (III) below. In particular, it is preferable to use the compound represented by Formula (III) below as the additive.

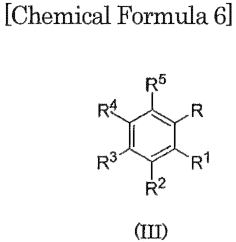

In Formula (III) above,
R is COOH or OH, and
R¹, R², R³, R⁴, and R⁵ are each independently selected from the group consisting of hydrogen, a halogen atom, NO₂, OH, COOH, NR⁶R⁷ (wherein R⁶ and R⁷ are each independently hydrogen, an alkyl group with 1 or more and 3 or less of carbon atoms, or an alkyl group with 1 or more and 3 or less of carbon atoms substituted with OH), an alkyl group with 1 or more and 6 or less of carbon atoms, an alkoxy group with 1 or more and 6 or less of carbon atoms, and a phenyl group (which is optionally substituted), or
two of R¹, R², R³, R⁴, and R⁵, taken together with the carbon atoms carrying them, form a saturated or unsaturated hydrocarbon ring, and the other three are each independently selected from the group consisting of hydrogen, a halogen atom, NO₂, OH, COOH, NR⁶R⁷ (wherein R⁶ and R⁷ are each independently hydrogen, an alkyl group with 1 or more and 3 or less of carbon atoms, or an alkyl group with 1 or more and 3 or less of carbon atoms substituted with OH), an alkyl group with 1 or more and 6 or less of carbon atoms, an alkoxy group with 1 or more and 6 or less of carbon atoms, and a phenyl group (which is optionally substituted).

In Formula (III) above, the alkyl group with 1 or more and 6 or less of carbon atoms is preferably an alkyl group with 1 or more and 4 or less of carbon atoms, and more preferably an alkyl group with 1 or 2 carbon atoms.

In Formula (III) above, the alkyl group with 1 or more and 3 or less of carbon atoms is preferably an alkyl group with 1 or 2 carbon atoms, and more preferably an alkyl group with 1 carbon atom.

In Formula (III) above, the alkoxy group with 1 or more and 6 or less of carbon atoms is preferably an alkoxy group with 1 or more and 4 or less of carbon atoms, and more preferably an alkoxy group with 1 or 2 carbon atoms.

In Formula (III) above, the halogen atom may be fluorine, chlorine, bromine, or iodine, and is preferably chlorine or bromine, and more preferably chlorine.

In Formula (III) above, the saturated or unsaturated hydrocarbon ring may be, for example, a saturated cyclic hydrocarbon with 3 or more and 10 or less of carbon atoms or an unsaturated cyclic hydrocarbon with 4 or more and 10 or less of carbon atoms.

In Formula (III) above, substituents of the phenyl group (which is optionally substituted) may be, for example, a halogen atom, OH, COOH, or a methyl group. The substituted phenyl group may be, for example, 2-chlorophenyl group, 3-chlorophenyl group, 4-chlorophenyl group, 2-bromophenyl group, 3-bromophenyl group, 4-bromophenyl group, 2-hydroxyphenyl group, 3-hydroxyphenyl group, 4-hydroxyphenyl group, 2-methylphenyl group, 3-methylphenyl group, 4-methylphenyl group, 2-carboxyphenyl group, 3-carboxyphenyl group, 4-carboxyphenyl group, 3-carboxy-4-hydroxyphenyl group, or 2-hydroxy-3-carboxyphenyl group, and preferably 3-carboxy-4-hydroxyphenyl group or 2-hydroxy-3-carboxyphenyl group.

In Formula (III) representing the additive,
it is preferable that R is COOH, and
   R¹, R², R³, R⁴, and R⁵ are each independently selected from the group consisting of hydrogen, a halogen atom, NO₂, COOH, OH, and an alkoxy group with 1 or more and 6 or less of carbon atoms,
it is more preferable that R is COOH, and
   one of R¹, R², R³, R⁴, and R⁵ is OH, and the others are each independently selected from the group consisting of hydrogen, a halogen atom, NO₂, and an alkoxy group with 1 or more and 6 or less of carbon atoms,
it is even more preferable that R is COOH,
   R¹ is OH, and R², R³, R⁴, and R⁵ are each independently selected from the group consisting of hydrogen, a halogen atom, NO₂, and an alkoxy group with 1 or more and 6 or less of carbon atoms (in this case, represented by Formula (ix) below),
it is even more preferable that R is COOH, and
   R² is OH, and R¹, R³, R⁴, and R⁵ are each independently selected from the group consisting of hydrogen, a halogen atom, NO₂, and an alkoxy group with 1 or more and 6 or less of carbon atoms (in this case, represented by Formula (x) below), and
it is even more preferable that R is COOH, and
   R³ is OH, and R¹, R², R⁴, and R⁵ are each independently selected from the group consisting of hydrogen, a halogen atom, NO₂, and an alkoxy group with 1 or more and 6 or less of carbon atoms (in this case, represented by Formula (xi) below).

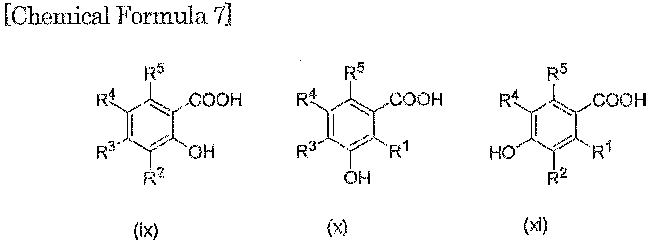

In Formula (ix) representing the additive,
it is more preferable that R² is hydrogen, a halogen atom, or an alkoxy group with 1 or more and 6 or less of carbon atoms,
R³ is hydrogen, a halogen atom, or NO₂,
R⁴ is hydrogen, a halogen atom, or NO₂, and
R⁵ is hydrogen. The additive represented by Formula (xii) is more preferably represented by, for example, Formulas (101) to (108) below.

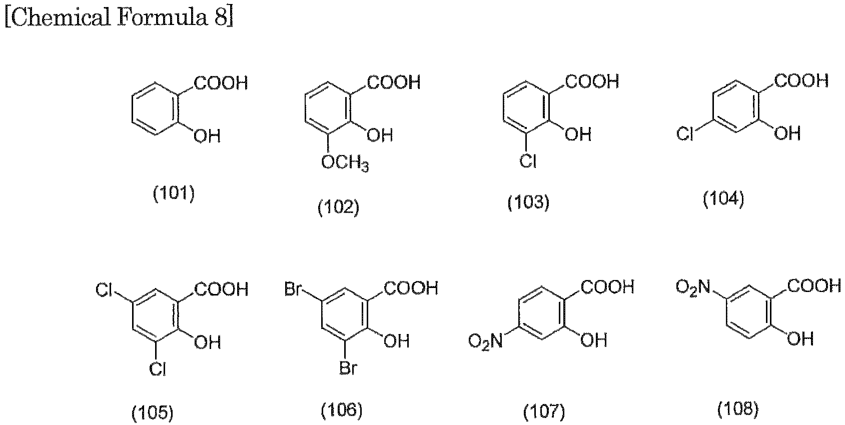

The additive of Formula (III) in which R is COOH, and R¹, R², R³, R⁴, and R⁵ are each independently selected from the group consisting of hydrogen, a halogen atom, NO₂, OH, COOH, NR⁶R⁷ (wherein R⁶ and R⁷ are each independently hydrogen, an alkyl group with 1 or more and 3 or less of carbon atoms, or an alkyl group with 1 or more and 3 or less of carbon atoms substituted with OH), an alkyl group with 1 or more and 6 or less of carbon atoms, an alkoxy group with 1 or more and 6 or less of carbon atoms, and a phenyl group (which is optionally substituted) may be, for example, benzoic acid, o-chlorobenzoic acid, m-chlorobenzoic acid, 2,4-dichlorobenzoic acid, 3,5-dichlorobenzoic acid, 3,5-dibromobenzoic acid, p-methylbenzoic acid, p-tert-butylbenzoic acid, p-hydroxybenzoic acid, m-hydroxybenzoic acid, 2,5-dihydroxybenzoic acid, 2,4-dihydroxybenzoic acid, 3,5-dihydroxybenzoic acid, 2,6-dihydroxybenzoic acid, 3,4-dihydroxybenzoic acid, 3-chloro-4-methoxybenzoic acid, 3-bromo-4-methoxybenzoic acid, phthalic acid, isophthalic acid, terephthalic acid, 3-fluorophthalic acid, 3-chlorophthalic acid, 3-bromophthalic acid, 3-methoxyphthalic acid, 3-methylphthalic acid, 4-fluorophthalic acid, 4-chlorophthalic acid, 4-bromophthalic acid, 4-methoxyphthalic acid, 4-methylphthalic acid, trimesic acid, salicylic acid, 3-methylsalicylic acid, 4-methylsalicylic acid, 5-methylsalicylic acid, 6-methylsalicylic acid, 3-ethylsalicylic acid, 4-ethylsalicylic acid, 5-ethylsalicylic acid, 6-ethylsalicylic acid, 3-isopropylsalicylic acid, 4-isopropylsalicylic acid, 5-isopropylsalicylic acid, 6-isopropylsalicylic acid, 3-tert-butylsalicylic acid, 4-tert-butylsalicylic acid, 5-tert-butylsalicylic acid, 6-tert-butylsalicylic acid, 3-fluorosalicylic acid, 4-fluorosalicylic acid, 5-fluorosalicylic acid, 6-fluorosalicylic acid, 4-fluorosalicylic acid, 5-fluorosalicylic acid, 3-chlorosalicylic acid, 4-chlorosalicylic acid, 5-chlorosalicylic acid, 6-chlorosalicylic acid, 3-bromosalicylic acid, 4-bromosalicylic acid, 5-bromosalicylic acid, 6-bromosalicylic acid, 3-iodosalicylic acid, 4-iodosalicylic acid, 5-iodosalicylic acid, 6-iodosalicylic acid, 3-nitrosalicylic acid, 4-nitrosalicylic acid, 5-nitrosalicylic acid, 6-nitrosalicylic acid, 3-methoxysalicylic acid, 4-methoxysalicylic acid, 5-methoxysalicylic acid, 6-methoxysalicylic acid, 3-ethoxysalicylic acid, 4-ethoxysalicylic acid, 5-ethoxysalicylic acid, 6-ethoxysalicylic acid, 3,5-difluorosalicylic acid, 3,5-dichlorosalicylic acid, 3,5-dibromosalicylic acid, 3,5-diiodosalicylic acid, 3,5-dimethoxysalicylic acid, 3-phenylsalicylic acid, biphenyl-2-carboxylic acid, or biphenyl-3-carboxylic acid, and is preferably selected from the group consisting of benzoic acid, 3,5-dichlorobenzoic acid, 3,5-dibromobenzoic acid, 3-chloro-4-methoxybenzoic acid, 3-bromo-4-methoxybenzoic acid, salicylic acid, 3-chlorosalicylic acid, 4-chlorosalicylic acid, 5-chlorosalicylic acid, 3-bromosalicylic acid, 4-bromosalicylic acid, 5-bromosalicylic acid, 3-methoxysalicylic acid, 4-methoxysalicylic acid, 5-methoxysalicylic acid, 3,5-dichlorosalicylic acid, 3,5-dibromosalicylic acid, 3-chloro-5-bromosalicylic acid, 3-bromo-5-chlorosalicylic acid, 4-nitrosalicylic acid, and 5-nitrosalicylic acid. In Formula (III), preferably one to three of R¹, R², R³, R⁴, and R⁵ are a halogen atom, and more preferably one or two of them are a halogen atom. In Formula (III), preferably one or two of R¹, R², R³, R⁴, and R⁵ are a nitro group and/or an alkoxy group with 1 or more and 6 or less of carbon atoms, and more preferably one of them is a nitro group and/or an alkoxy group with 1 or more and 6 or less of carbon atoms.

The additive of Formula (III) is preferably benzenecarboxylic acid (benzoic acid), benzenedicarboxylic acid (including phthalic acid, isophthalic acid, terephthalic acid), or benzenehydroxycarboxylic acid (including salicylic acid), which is optionally substituted with a halogen atom, NO₂, or an alkoxy group with 1 or more and 6 or less of carbon atoms, and more preferably salicylic acid that is optionally substituted with a halogen atom, NO₂, or an alkoxy group with 1 or more and 6 or less of carbon atoms.

In Formula (III) representing the additive, it is preferable that R is COOH, one of R¹, R², R³, R⁴, and R⁵ is COOH, and the others are each independently selected from the group consisting of hydrogen, a halogen atom, NO₂, OH, COOH, NR⁶R⁷ (wherein R⁶ and R⁷ are each independently hydrogen, an alkyl group with 1 or more and 3 or less of carbon atoms, or an alkyl group with 1 or more and 3 or less of carbon atoms substituted with OH), an alkyl group with 1 or more and 6 or less of carbon atoms, an alkoxy group with 1 or more and 6 or less of carbon atoms, and a phenyl group (which is optionally substituted). Examples of such an additive include phthalic acid, isophthalic acid, terephthalic acid, 3-fluorophthalic acid, 3-chlorophthalic acid, 3-bromophthalic acid, 3-methoxyphthalic acid, 3-methylphthalic acid, 4-fluorophthalic acid, 4-chlorophthalic acid, 4-bromophthalic acid, 4-methoxyphthalic acid, 4-methylphthalic acid, and trimesic acid.

In Formula (III) representing the additive, it is preferable that two of R¹, R², R³, R⁴, and R⁵, taken together with the carbon atoms carrying them, form an unsaturated hydrocarbon ring, and the other three are each independently selected from the group consisting of hydrogen, a halogen atom, NO₂, OH, COOH, NR⁶R⁷ (wherein R⁶ and R⁷ are each independently hydrogen, an alkyl group with 1 or more and 3 or less of carbon atoms, or an alkyl group with 1 or more and 3 or less of carbon atoms substituted with OH), an alkyl group with 1 or more and 6 or less of carbon atoms, an alkoxy group with 1 or more and 6 or less of carbon atoms, and a phenyl group (which is optionally substituted). Examples of such an additive include 1-naphthoic acid and 2-naphthoic acid.

In Formula (III) representing the additive, it is preferable that R is OH, and R¹, R², R³, R⁴, and R⁵ are each independently selected from the group consisting of hydrogen and NO₂. Examples of the additive of Formula (III) in which R is OH and R¹, R², R³, R⁴, and R⁵ are each independently selected from the group consisting of hydrogen and NO₂ include o-nitrophenol, m-nitrophenol, and p-nitrophenol.

The polycarboxylic acid is preferably aliphatic dicarboxylic acid such as oxalic acid, malonic acid, succinic acid, fumaric acid, maleic acid, or adipic acid. The number of carbon atoms in the polycarboxylic acid is preferably 2 or more, and is preferably 10 or less, and more preferably 6 or less.

The molar ratio of the additive to the compound represented by General Formula (II) [additive / compound represented by General Formula (II)] is, for example, 0.001 or more, preferably 0.01 or more, and more preferably 0.05 or more, from the viewpoint of improving the reaction yield, and is preferably 10 or less, 1 or less, or 0.5 or less, from the viewpoint of reducing the production cost. The molar ratio between the compound represented by General Formula (II) and the additive [compound represented by General Formula (II) : additive] is, for example, 0.001 or more and 10 or less, preferably 0.01 or more and 1 or less, and more preferably 0.05 or more and 0.5 or less, from the viewpoint of improving the reaction yield.

The molar ratio of the additive to the metal catalyst [additive / metal catalyst] is, for example, 0.01 or more, preferably 0.5 or more, and more preferably 1 or more, from the viewpoint of improving the reaction yield, and is, for example, 100 or less, preferably 30 or less, and more preferably 10 or less, from the viewpoint of reducing the production cost. The molar ratio of the additive to the metal catalyst [additive / metal catalyst] is, for example, 0.01 or more and 100 or less, preferably 0.5 or more and 30 or less, and more preferably 1 or more and 10 or less, from the viewpoint of improving the reaction yield.

The reaction temperature in the method of the present invention is generally 0°C or higher and 100°C or lower, and, from the viewpoint of safely improving the reaction yield, the temperature is preferably 20°C or higher, and is preferably 80°C or lower, and more preferably 50°C or lower.

The reaction pressure in the method of the present invention is not particularly limited, but the reaction is generally performed under atmospheric pressure. In the method of the present invention, the pressure may be increased or reduced as needed.

The reaction time in the method of the present invention depends on the yield, but is preferably 1 hour or longer, and more preferably 10 hours or longer, and is preferably 100 hours or shorter, and more preferably 60 hours or shorter.

The reaction in the method of the present invention may be performed in the presence of an organic solvent. The organic solvent is not particularly limited, as long as it is a solvent that makes the reaction solution homogeneous. The organic solvent can be ethers or alcohols, and, from the viewpoint of reaction yield, the organic solvent is preferably alcohols, more preferably saturated aliphatic alcohols, and even more preferably t-butyl alcohol.

In the method of the present invention, the mass ratio of the solvent to the compound of Formula (II) [solvent / compound of Formula (II)] is, for example, 1 or more, preferably 2 or more, and more preferably 3 or more, from the viewpoint of improving the reaction yield, and is, for example, 30 or less, preferably 10 or less, more preferably 7 or less, and even more preferably 5 or less, from the viewpoint of reducing the production cost. In the method of the present invention, the mass ratio of the solvent to the compound of Formula (II) [solvent / compound of Formula (II)] is, for example, 1 or more and 30 or less, preferably 1 or more and 10 or less, more preferably 2 or more and 7 or less, and even more preferably 3 or more and 5 or less, from the viewpoint of improving the reaction yield.

Step of Obtaining Compound Represented by General Formula (I) through Oxidative Cleavage of Compound Represented by General Formula (II) using Oxidant in Presence of Metal Catalyst Containing One or More Metal Elements Selected from Group Consisting of Vanadium, Iron, and Molybdenum

In this step, for example, the reaction is performed by mixing the metal catalyst, the oxidant, the compound represented by General Formula (II), the organic solvent, and optionally the additive in a reactor at a predetermined temperature typically under stirring. The order of addition of the metal catalyst, the oxidant, the compound represented by General Formula (II), the organic solvent, and the optional additive at this time is not particularly limited, but, from the viewpoint of safety, the reaction may be performed by continuously adding the oxidant dropwise to a mixed solution of the metal catalyst, the compound represented by General Formula (II), the optional additive, and organic solvent. Alternatively, a catalyst solution may be prepared by mixing the metal catalyst, the optional additive, and the oxidant in advance, and then the catalyst solution may be added to a mixed solution of the compound represented by General Formula (II) and the organic solvent to perform the reaction.

After the reaction is completed, for example, oxides remaining in the reaction system can be decomposed by a reducing agent, for example, and then the compound represented by General Formula (I) can be purified through separation of oil and water layers, solvent evaporation, distillation, silica gel column chromatography, or the like.

The present invention further provides a method for producing a cyclic ketone compound represented by Formula (3). The cyclic ketone compound represented by Formula (3) is a mixture of 3-methyl-4-cyclopentadecen-1-one and 3-methyl-5-cyclopentadecen-1-one, and is known as muscenone (manufactured by Firmenich). The cyclic ketone compound represented by Formula (3), which is a musk fragrance material, is a cyclic compound with excellent biodegradability, as well as high residual fragrance and elegant texture. There is a need to develop a production method with high production safety and excellent production efficiency that meets the growing needs for synthetic musk fragrances in recent years. The method for producing a cyclic ketone compound represented by Formula (3) using 3-methyl-1,5-cyclopentadecanedione (1) obtained by the method of the present invention has any one of Steps (a), (b), and (c) below.

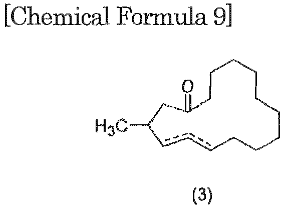

Step (a) of obtaining a cyclic ketone compound represented by Formula (3) through partial reduction, followed by dehydration of 3-methyl-1,5-cyclopentadecanedione,

Step (b) of obtaining muscenone through reduction, followed by enol etherification, and further followed by ring opening of 3-methyl-1,5-cyclopentadecanedione, or

Step (c) of obtaining muscenone through partial reduction and enol etherification, followed by ring opening of 3-methyl-1,5-cyclopentadecanedione.

Step (a) includes, for example,
Step (a-1) of obtaining 3-methylcyclopentadecanol-5-one (2) through partial reduction of 3-methyl-1,5-cyclopentadecanedione (1), and
Step (a-2) of obtaining a cyclic ketone compound represented by Formula (3) through dehydration of 3-methylcyclopentadecanol-5-one (2).

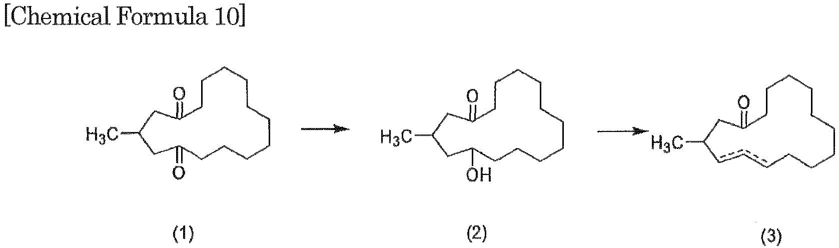

Step (b) includes, for example,
Step (b-1) of obtaining 3-methylcyclopentadecane-1,5-diol (4) through reduction of 3-methyl-1,5-cyclopentadecanedione (1),
Step (b-2) of obtaining 16-oxa-3-methylbicyclo[10.3.1]pentadec-1-ene (5) through partial oxidation and enol etherification of 3-methylcyclopentadecane-1,5-diol (4), and
Step (b-3) of obtaining a cyclic ketone compound represented by Formula (3) through ring opening of 16-oxa-3-methylbicyclo[10.3.1]pentadec-1-ene (5).

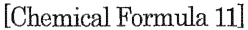

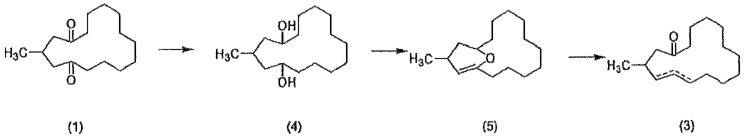

Step (c) includes, for example,
Step (c-1) of obtaining 16-oxa-3-methylbicyclo[10.3.1]pentadec-1-ene (5) through partial reduction and enol etherification of 3-methyl-1,5-cyclopentadecanedione (1), and
Step (c-2) of obtaining a cyclic ketone compound represented by Formula (3) through ring opening of 16-oxa-3-methylbicyclo[10.3.1]pentadec-1-ene (5).

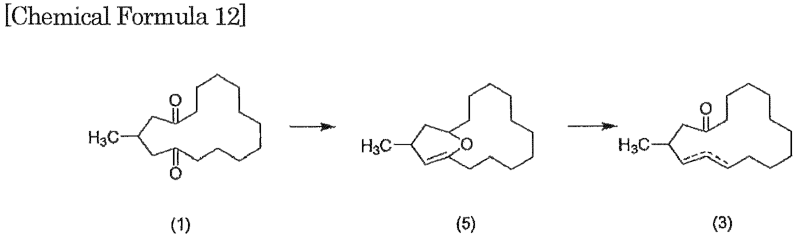

The above-mentioned 3-methyl-1,5-cyclopentadecanedione (1) is obtained using the method for producing a compound represented by Formula (I) of the present invention.

First, a method for producing a cyclic ketone compound represented by Formula (3) including Step (a) will be described.

Step (a) includes
Step (a-1) of obtaining 3-methylcyclopentadecanol-5-one (2) through partial reduction of 3-methyl-1,5-cyclopentadecanedione (1), and
Step (a-2) of obtaining a cyclic ketone compound represented by Formula (3) through dehydration of 3-methylcyclopentadecanol-5-one (2).

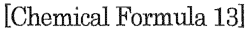

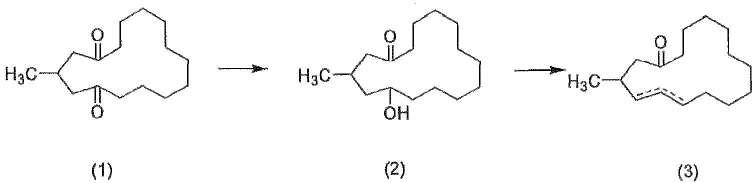

The partial reduction of 3-methyl-1,5-cyclopentadecanedione (1) in Step (a-1) above can be performed according to a method using metal hydrides such as sodium borohydride or sodium hydride, or a method of performing hydrogenation in the presence of a metal catalyst. From the viewpoint of safety and reaction yield, the partial reduction is preferably performed according to a method of performing hydrogenation in the presence of a metal catalyst.

The metal catalyst in the hydrogenation method can be palladium on carbon, Raney nickel, or the like. From the viewpoint of allowing hydrogenation to be performed under mild reaction conditions, the metal catalyst in the hydrogenation method is preferably Raney nickel.

The amount of metal catalyst that is used for 3-methyl-1,5-cyclopentadecanedione (1) in Step (a-1) above is such that the molar ratio between 3-methyl-1,5-cyclopentadecanedione (1) and the metal catalyst [3-methyl-1,5-cyclopentadecanedione (1) : metal catalyst] is preferably 1:0.01 to 1:1, and more preferably 1:0.1 to 1:0.5, from the viewpoint of reaction yield and cost.

The reaction temperature in Step (a-1) above is preferably 0°C or higher from the viewpoint of improving the reaction yield, and is preferably 100°C or lower, more preferably 50°C or lower, and even more preferably 30°C or lower, from the viewpoint of suppressing side reactions.

The reaction time in Step (a-1) above is preferably 0.1 hour or longer, and more preferably 0.5 hours or longer, and is preferably 10 hours or shorter, more preferably 3 hours or shorter, and even more preferably 1 hour or shorter from the viewpoint of improving the reaction yield.

The hydrogen pressure in the hydrogenation is preferably 0.1 MPa or higher, and is preferably 2.0 MPa or lower, and more preferably 1.0 MPa or lower, from the viewpoint of safety and reaction selectivity.

The reaction of Step (a-1) above may be performed in the presence of an organic solvent. The organic solvent is not particularly limited, as long as it is a solvent in which the reaction substrate dissolves. The organic solvent can be alcohols, and, from the viewpoint of production efficiency and cost, the organic solvent is preferably lower alcohols with 1 or more and 3 or less of carbon atoms.

The dehydration of 3-methylcyclopentadecanol-5-one (2) in Step (a-2) above can be performed according to a method of performing dehydration in the presence of an acid.

Examples of the acid for use in Step (a-2) above include sulfuric acid, phosphoric acid, and benzenesulfonic acid, and, from the viewpoint of equipment load and reaction yield, the acid is preferably benzenesulfonic acid.

The amount of acid that is used for 3-methylcyclopentadecanol-5-one (2) in Step (a-2) above is such that the molar ratio between 3-methylcyclopentadecanol-5-one (2) and the acid [3-methylcyclopentadecanol-5-one (2) : acid] is preferably 1:0.01 to 1:1, and more preferably 1:0.05 to 1:0.3, from the viewpoint of production cost and reaction yield.

The reaction temperature in Step (a-2) above is preferably 20°C or higher, more preferably 50°C or higher, and even more preferably 70°C or higher, from the viewpoint of improving the reaction yield, and is preferably 200°C or lower, more preferably 150°C or lower, and even more preferably 130°C or lower, from the viewpoint of safety.

The reaction time in Step (a-2) above is preferably 0.25 hours or longer, and more preferably 0.5 hours or longer, and is preferably 20 hours or shorter, more preferably 10 hours or shorter, and even more preferably 3 hours or shorter from the viewpoint of improving the reaction yield.

The reaction of Step (a-2) above may be performed in the presence of an organic solvent. The organic solvent is not particularly limited, as long as it is a solvent that azeotropes with water. The organic solvent can be hydrocarbons, and, from the viewpoint of dehydration efficiency, the organic solvent is preferably toluene.

Next, a method for producing a cyclic ketone compound represented by Formula (3) including Step (b) will be described.

Step (b) includes
Step (b-1) of obtaining 3-methylcyclopentadecane-1,5-diol (4) through reduction of 3-methyl-1,5-cyclopentadecanedione (1),
Step (b-2) of obtaining 16-oxa-3-methylbicyclo[10.3.1]pentadec-1-ene (5) through partial oxidation and enol etherification of 3-methylcyclopentadecane-1,5-diol (4), and
Step (b-3) of obtaining a cyclic ketone compound represented by Formula (3) through ring opening of 16-oxa-3-methylbicyclo[10.3.1]pentadec-1-ene (5).

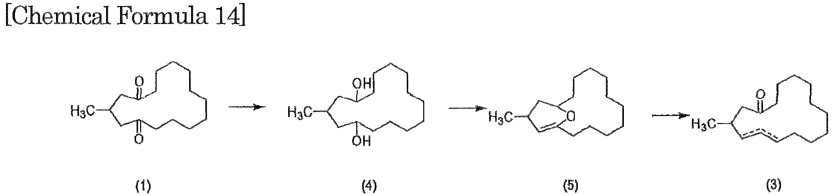

The partial reduction of 3-methyl-1,5-cyclopentadecanedione (1) in Step (b-1) above can be performed according to a method using metal hydrides such as sodium borohydride or sodium hydride, or a method of performing hydrogenation in the presence of a metal catalyst. From the viewpoint of safety and reaction yield, the partial reduction is preferably performed according to a method of performing hydrogenation in the presence of a metal catalyst.

The metal catalyst in the hydrogenation method can be palladium on carbon, Raney nickel, or the like. From the viewpoint of allowing hydrogenation to be performed under mild reaction conditions, the metal catalyst is preferably Raney nickel.

The amount of metal catalyst that is used for 3-methyl-1,5-cyclopentadecanedione (1) in Step (b-1) above is such that the molar ratio between 3-methyl-1,5-cyclopentadecanedione (1) and the metal catalyst [3-methyl-1,5-cyclopentadecanedione (1) : metal catalyst] is preferably 1:0.01 to 1:1, and more preferably 1:0.1 to 1:0.5, from the viewpoint of reaction yield and cost.

The reaction temperature in Step (b-1) above is preferably 0°C or higher from the viewpoint of improving the reaction yield, and is preferably 100°C or lower, more preferably 50°C or lower, and even more preferably 30°C or lower, from the viewpoint of suppressing side reactions.

The reaction time in Step (b-1) above is preferably 1 hour or longer, more preferably 5 hours or longer, and even more preferably 10 hours or longer, and is preferably 30 hours or shorter, and more preferably 25 hours or shorter from the viewpoint of improving the reaction yield.

The hydrogen pressure in the hydrogenation is preferably 0.1 MPa or higher, and is preferably 2.0 MPa or lower, and more preferably 1.0 MPa or lower, from the viewpoint of safety and reaction selectivity.

The reaction of Step (b-1) above may be performed in the presence of an organic solvent. The organic solvent is not particularly limited, as long as it is a solvent in which the reaction substrate dissolves. The organic solvent can be alcohols, and, from the viewpoint of production efficiency and cost, the organic solvent is preferably lower alcohols with 1 or more and 3 or less of carbon atoms.

The partial oxidation and enol etherification of 3-methylcyclopentadecane-1,5-diol (4) in Step (b-2) above can be performed according to a method of performing partial oxidation and enol etherification in the presence of a metal catalyst.

The metal catalyst for use in Step (b-2) above can be a metal catalyst with oxidation capability, such as aluminum oxide, iron oxide, or Raney copper, and, from the viewpoint of improving the reaction yield, the metal catalyst is preferably Raney copper.

The amount of metal catalyst that is used for 3-methylcyclopentadecane-1,5-diol (4) in Step (b-2) above is such that the molar ratio between 3-methylcyclopentadecane-1,5-diol (4) and the metal catalyst [3-methylcyclopentadecane-1,5-diol (4) : metal catalyst] is preferably 1:0.01 to 1:1 from the viewpoint of reaction yield and cost, and more preferably 1:0.05 to 1:0.3 from the viewpoint of shortening the reaction time and reducing waste.

The reaction temperature in Step (b-2) above is preferably 100°C or higher, and more preferably 120°C or higher, from the viewpoint of improving the reaction yield, and is preferably 300°C or lower, more preferably 200°C or lower, and even more preferably 180°C or lower, from the viewpoint of suppressing side reactions.

The reaction time in Step (b-2) above is preferably 1 hour or longer, and more preferably 2 hours or longer, from the viewpoint of improving the reaction yield, and is preferably 20 hours or shorter, more preferably 10 hours or shorter, and even more preferably 5 hours or shorter, from the viewpoint of improving the productivity.

The ring opening of 16-oxa-3-methylbicyclo[10.3.1]pentadec-1-ene (5) in Step (b-3) above can be performed according to a method of performing ring opening in the presence of an acid.

Examples of the acid for use in Step (b-3) above include sulfuric acid, phosphoric acid, and benzenesulfonic acid, and, from the viewpoint of ease of handling and reaction yield, the acid is preferably phosphoric acid.

The amount of acid that is used for 16-oxa-3-methylbicyclo[10.3.1]pentadec-1-ene (5) in Step (b-3) above is such that the molar ratio between 16-oxa-3-methylbicyclo[10.3.1]pentadec-1-ene (5) and the acid [16-oxa-3-methylbicyclo[10.3.1]pentadec-1-ene (5) : acid] is preferably 1:0.01 to 1:1, and more preferably 1:0.1 to 1:0.5, from the viewpoint of production efficiency and reaction yield.

The reaction temperature in Step (b-3) above is preferably 20°C or higher, more preferably 50°C or higher, and even more preferably 70°C or higher, from the viewpoint of improving the reaction yield, and is preferably 200°C or lower, more preferably 150°C or lower, and even more preferably 130°C or lower, from the viewpoint of suppressing side reactions.

The reaction time in Step (b-3) above is preferably 0.5 hours or longer, and more preferably 1 hour or longer, and is preferably 20 hours or shorter, more preferably 10 hours or shorter, and even more preferably 5 hours or shorter from the viewpoint of improving the reaction yield.

The reaction of Step (b-3) above may be performed in the presence of an organic solvent. The organic solvent is not particularly limited, as long as it is a solvent that azeotropes with water. The organic solvent can be hydrocarbons, and, from the viewpoint of dehydration efficiency, the organic solvent is preferably toluene.

Next, a method for producing a cyclic ketone compound represented by Formula (3) including Step (c) will be described.

This step includes
Step (c-1) of obtaining 16-oxa-3-methylbicyclo[10.3.1]pentadec-1-ene (5) through partial reduction and enol etherification of 3-methyl-1,5-cyclopentadecanedione (1), and
Step (c-2) of obtaining a cyclic ketone compound represented by Formula (3) through ring opening of 16-Oxa-3-methylbicyclo[10.3.1]pentadec-1-ene (5).

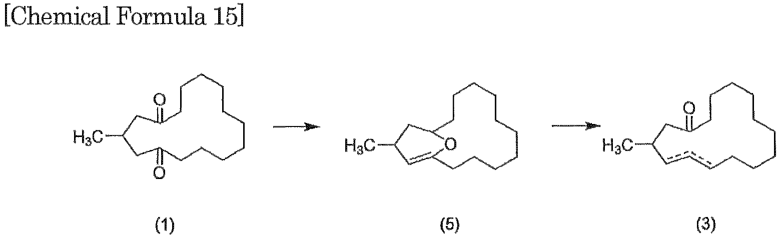

The partial reduction and enol etherification of 3-methyl-1,5-cyclopentadecanedione (1) in Step (c-1) above can be performed according to a method of performing the reaction in the presence of a metal catalyst containing one or more metal elements selected from the group consisting of magnesium, aluminum, zirconium, titanium, and samarium and an alcohol containing one or more selected from the group consisting of primary alcohol and secondary alcohol.

The metal catalyst is a metal catalyst containing one or more metal elements selected from the group consisting of magnesium, aluminum, zirconium, titanium, and samarium. The metal catalyst preferably contains one or more metal elements selected from the group consisting of aluminum, zirconium, and titanium. The metal catalyst may be aluminum alkoxide, zirconium alkoxide, titanium alkoxide, zirconium oxide, or the like. The aluminum alkoxide may be aluminum ethoxide, aluminum isopropoxide, aluminum n-butoxide, aluminum sec-butoxide, aluminum tert-butoxide, or the like. The catalyst containing zirconium may be zirconia (zirconium dioxide) and zirconium alkoxide. The zirconium alkoxide may be zirconium(IV) ethoxide, zirconium(IV) isopropoxide, zirconium(IV) n-propoxide, zirconium(IV) n-butoxide, zirconium(IV) sec-butoxide, zirconium(IV) tert-butoxide, or the like. The metal catalyst is preferably aluminum alkoxide, zirconium alkoxide, or titanium alkoxide, and more preferably aluminum alkoxide or zirconium alkoxide, because of their high catalytic activity (i.e., high reaction rate and high reaction yield). The metal catalyst is preferably zirconium alkoxide, and more preferably zirconium(IV) n-butoxide, zirconium sec-butoxide, or zirconium tert-butoxide, because the amount used can be reduced to a catalyst amount. The metal catalyst is preferably zirconium(IV) n-butoxide because of its industrial availability.

The alcohol is an alcohol containing one or more selected from the group consisting of primary alcohol and secondary alcohol. The primary alcohol may be ethanol, 1-propanol, 1-butanol, 2-methylpropanol, 1-pentanol, 2-methylbutanol, 3-methylbutanol, 1-hexanol, 2-methylpentanol, 3-methylpentanol, 2-ethylbutanol, 1-heptanol, 2-methylhexanol, 1-octanol, 1-nonanol, 1-decanol, or the like. The secondary alcohol is not limited in valency, and is not particularly limited, as long as it has one or more secondary hydroxyl groups. The secondary alcohol may have linear, branched, or cyclic aliphatic groups, aromatic groups, or a combination of both. Examples of the secondary alcohol include isopropyl alcohol, 2-butanol, 2-pentanol, 3-pentanol, cyclopentanol, 2-hexanol, 3-hexanol, cyclohexanol, 3-methyl-2-pentanol, 4-methyl-2-pentanol, 2-heptanol, 3-heptanol, 4-heptanol, 3-methyl-2-hexanol, 4-methyl-2-hexanol, 5-methyl-2-hexanol, cycloheptanol, 2-octanol, 3-octanol, 4-octanol, cyclooctanol, 2-nonanol, 3-nonanol, 4-nonanol, and 5-nonanol. The secondary alcohol is preferably cyclohexanol or 2-octanol because the reaction rate can be increased by raising the temperature.

The alcohol is preferably secondary alcohol. The alcohol is preferably an alcohol with a boiling point of 100°C or higher because the reaction selectivity can be improved and the reaction time can be shortened due to the reaction proceeding while removing by-product water from the system. Furthermore, the alcohol is preferably an alcohol with a boiling point of 200°C or lower because the boiling point is moderate and excessive reaction progress does not occur during azeotropic boiling of water, as a result of which the reaction selectivity is prevented from lowering. The alcohol is preferably an alcohol with a boiling point of 150°C or higher and 200°C or lower because the reaction rate can be increased by raising the temperature. The boiling points are specifically 82.4°C for isopropanol, 161.8°C for cyclohexanol, 100°C for 2-butanol, and 174°C for 2-octanol. The alcohol containing one or more selected from the group consisting of primary alcohol and secondary alcohol is preferably 2-octanol or cyclohexanol because of their industrial availability.

### Reaction Temperature

In the present invention, the step of obtaining a compound of Formula (5) through reaction of the compound of Formula (1) in the presence of the metal catalyst and the alcohol is performed at, for example, 80°C or higher, preferably 100°C or higher, and more preferably 150°C or higher, and at, for example, 280°C or lower, preferably 250°C or lower, and more preferably 190°C or lower.

### Reaction Time

In the present invention, the reaction time of the step of obtaining a compound of Formula (5) through reaction of the compound of Formula (1) in the presence of the metal catalyst and the alcohol is, for example, 2 hours to 5 days, preferably 4 hours to 2 days, and more preferably 6 hours to 24 hours from the viewpoint of production cost and production efficiency.

### LoadingAmount

In the present invention, in the step of obtaining a compound of Formula (5) through reaction of the compound of Formula (1) in the presence of the metal catalyst and the alcohol, the molar ratio between the compound of Formula (1) and the alcohol [compound of Formula (1) : alcohol] is, for example, 1:1 to 1:100, preferably 1:1 to 1:50, and more preferably 1:1.5 to 1:5 from the viewpoint of production cost and production efficiency.

In a specific implementation, the reaction is performed by mixing the metal catalyst, the alcohol, and the compound represented by General Formula (1) in a reactor at a predetermined temperature typically under stirring. At this time, the metal catalyst, the alcohol, and the compound represented by General Formula (1) may be added in any order, and an additive may be further added as needed. It is more preferable to perform the reaction in a state in which a Dean-Stark apparatus or a rectifying column is attached to the reactor. After the reaction is completed, the compound represented by General Formula (5) can be purified through distillation.

In the step of obtaining a compound of Formula (5) through reaction of the compound of Formula (1) in the presence of the metal catalyst and the alcohol, if the metal catalyst is a metal catalyst containing zirconium, the molar ratio between the compound of Formula (1) and the metal catalyst [compound of Formula (5) : metal catalyst] is, for example, 1:0.001 to 1:1.5, preferably 1:0.01 to 1:1.2, and more preferably 1:0.05 to 1:1.1 from the viewpoint of production cost. If the metal catalyst is a metal catalyst containing one or more metal elements selected from the group consisting of magnesium, aluminum, titanium, and samarium, the molar ratio between the compound of Formula (1) and the metal catalyst [compound of Formula (1) : metal catalyst] is, for example, 1:0.3 to 1:3, preferably 1:0.5 to 1:1.5, and more preferably 1:0.8 to 1:1.2 from the viewpoint of production cost.

### Additive

In the present invention, the step of obtaining a compound of Formula (5) through reaction of the compound of Formula (1) in the presence of the metal catalyst and the alcohol may be performed in the presence of an additive. Examples of the additive include an acid, a diol, a diamine, and an aminoalcohol. The acid may be, for example, phosphoric acid, sulfuric acid, para-toluenesulfonic acid, acetic acid, chloroacetic acid, trifluoroacetic acid (TFA), or the like. The diol may be, for example, ethylene glycol (EG), 1,2-cyclohexanediol, 1,3-propanediol, 1,4-butanediol, 1,5-pentanediol, 1,6-hexanediol, or the like. The diamine may be ethylenediamine, 1,2-diaminocyclohexane, 1,2-phenylenediamine, or the like. The aminoalcohol may be 2-aminoethanol, 3-aminopropanol, 2-aminopropanol, 1-amino-2-propanol, valinol, phenylalaninol, or the like. The additive is preferably an acid or a diol, and more preferably TFA or EG, from the viewpoint of shortening the reaction time. In the case of using an acid as the additive, the molar ratio between the metal catalyst and the acid [metal catalyst: acid] is, for example, 1:0.05 to 1:5, and preferably 1:1 to 1:3. In the case of using a diol as the additive, the molar ratio between the metal catalyst and the diol [metal catalyst: diol] is, for example, 1:0.1 to 1:5, and preferably 1:1 to 1:3. In the case of using an acid and a diol as the additive, the molar ratio between the metal catalyst and the acid and the diol [metal catalyst: acid: diol] is, for example, 1 : 0.1 to 5 : 0.1 to 5, and preferably 1:1 to 3 : 1 to 3.

The method including Step (c-2) of obtaining a cyclic ketone compound represented by Formula (3) through ring opening of 16-oxa-3-methylbicyclo[10.3.1]pentadec-1-ene (5) can be performed according to a method of performing ring opening in the presence of an acid, as with Step (b-3) above. The method of performing ring opening in the presence of an acid is as described in Step (b-3) above.

With respect to the foregoing embodiment, the present invention further discloses a method for producing a compound represented by General Formula (I) and a method for producing a cyclic ketone compound represented by Formula (3) as follows.
[1] A method for producing a compound represented by General Formula (I), including a step of obtaining a compound represented by General Formula (I) through oxidative cleavage of a compound represented by General Formula (II) using an oxidant in the presence of a metal catalyst containing one or more metal elements selected from the group consisting of vanadium, iron, and molybdenum. 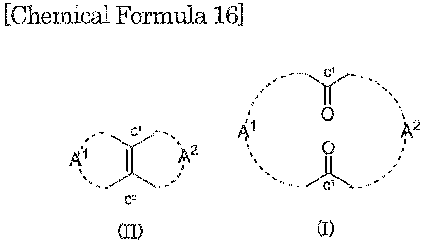 In Formulas (I) and (II) above,
   Formula -A¹- (wherein the former bonding hand means a bonding hand that is bonded to a carbon atom C¹, and the latter bonding hand means a bonding hand that is bonded to a carbon atom C²) is an alkylene group with 2 or more and 6 or less of carbon atoms that is optionally substituted and that optionally further contains an ether bond, an ester bond, a secondary amino group, a thioether group, or a combination thereof, and
   Formula -A²- (wherein the former bonding hand means a bonding hand that is bonded to a carbon atom C¹, and the latter bonding hand means a bonding hand that is bonded to a carbon atom C²) is an alkylene group with 4 or more and 10 or less of carbon atoms that is optionally substituted and that optionally further contains an ether bond, an ester bond, a secondary amino group, a thioether group, or a combination thereof.
[2] The production method according to [1],
   wherein Formula -A¹- is an alkylene group with 3 or 4 carbon atoms that is optionally substituted, and
   Formula -A²- is an alkylene group with 4, 6, 8 or 10 carbon atoms that is optionally substituted.
[3] The production method according to [1] or [2], wherein Formula -A¹- is a Formula -CH₂-CH(CH₃)-CH₂- group, and Formula -A²- is a Formula -(CH₂)₁₀- group.
[4] The production method according to any one of [1] to [3], wherein the oxidative cleavage uses a compound represented by Formula (III) below as an additive. 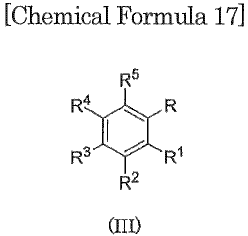 In Formula (III) above,
   R is COOH or OH, and
   R¹, R², R³, R⁴, and R⁵ are each independently selected from the group consisting of hydrogen, a halogen atom, NO₂, OH, COOH, NR⁶R⁷ (wherein R⁶ and R⁷ are each independently hydrogen, an alkyl group with 1 or more and 3 or less of carbon atoms, or an alkyl group with 1 or more and 3 or less of carbon atoms substituted with OH), an alkyl group with 1 or more and 6 or less of carbon atoms, an alkoxy group with 1 or more and 6 or less of carbon atoms, and a phenyl group (which is optionally substituted), or
   two of R¹, R², R³, R⁴, and R⁵, taken together with the carbon atoms carrying them, form a saturated or unsaturated hydrocarbon ring, and the other three are each independently selected from the group consisting of hydrogen, a halogen atom, NO₂, OH, COOH, NR⁶R⁷ (wherein R⁶ and R⁷ are each independently hydrogen, an alkyl group with 1 or more and 3 or less of carbon atoms, or an alkyl group with 1 or more and 3 or less of carbon atoms substituted with OH), an alkyl group with 1 or more and 6 or less of carbon atoms, an alkoxy group with 1 or more and 6 or less of carbon atoms, and a phenyl group (which is optionally substituted).
[5] The production method according to [3] or [4],
   wherein R is COOH, and
   R¹, R², R³, R⁴, and R⁵ are each independently selected from the group consisting of hydrogen, a halogen atom, NO₂, COOH, OH, and an alkoxy group with 1 or more and 6 or less of carbon atoms.
[6] The production method according to any one of [3] to [5], wherein R is COOH, one of R¹, R², R³, R⁴, and R⁵ is OH, and the others are each independently selected from the group consisting of hydrogen, a halogen atom, NO₂, and an alkoxy group with 1 or more and 6 or less of carbon atoms.
[7] The production method according to any one of [3] to [6], wherein the additive contains one or more selected from benzenecarboxylic acid, benzenedicarboxylic acid, and benzenehydroxycarboxylic acid that are optionally substituted with a halogen atom, NO₂, or an alkoxy group with 1 or more and 6 or less of carbon atoms, and preferably contains salicylic acid that is optionally substituted with a halogen atom, NO₂, or an alkoxy group with 1 or more and 6 or less of carbon atoms.
[8] The production method according to any one of [1] to [7], wherein the metal catalyst is one or more selected from a vanadic acid compound, a vanadate, a vanadium alkoxide compound, and a vanadium complex compound.
[9] The production method according to any one of [1] to [8], wherein the metal catalyst is one or more selected from a molybdic acid compound and a molybdenum complex compound.
[10] The production method according to any one of [1] to [9], wherein the metal catalyst is one or more selected from iron oxide, iron nitrate, iron sulfate, iron alkoxide, iron carboxylate, iron halide, and iron complex.
[11] The production method according to any one of [1] to [10], wherein the metal catalyst is selected from the group consisting of a vanadium(IV) compound, a vanadium(V) compound, a molybdenum(VI) compound, an iron(II) compound, and an iron(III) compound.
[12] The production method according to any one of [1] to [11], wherein the metal catalyst is selected from the group consisting of vanadium(V) oxide, vanadate(V), vanadium(V) oxytrialkoxide, and vanadium(IV) complex.
[13] The production method according to any one of [1] to [12], wherein the metal catalyst is selected from the group consisting of molybdenum(VI) oxide, molybdic acid, polymolybdic acid, heteropolymolybdic acid, and dioxomolybdenum(VI) complex.
[14] The production method according to any one of [1] to [13], wherein the metal catalyst is selected from the group consisting of iron(II) oxide, iron(III) oxide, iron(II) nitrate, iron(III) nitrate, iron(II) sulfate, iron(III) sulfate, iron alkoxide, iron carboxylate, iron halide, and iron complex.
[15] The production method according to any one of [1] to [14], wherein the oxidant is one or more selected from tert-butyl hydroperoxide, cumene hydroperoxide, di-tert-butyl peroxide, dimethyldioxirane, acetone peroxide, methyl ethyl ketone peroxide, hexamethylenetriperoxidediamine, hydrogen peroxide, lithium peroxide, sodium peroxide, potassium peroxide, and permanganate.
[16] The production method according to any one of [1] to [15], wherein the oxidant contains hydrogen peroxide or tert-butyl hydroperoxide.
[17] The production method according to any one of [1] to [16], wherein a molar ratio of the metal catalyst to the compound represented by General Formula (II) [metal catalyst / compound represented by General Formula (II)] is 0.0001 or more and 1 or less.
[18] The production method according to any one of [1] to [17], wherein a molar ratio of the metal catalyst to the compound represented by General Formula (II) [metal catalyst / compound represented by General Formula (II)] is 0.001 or more and 0.7 or less.
[19] The production method according to any one of [1] to [18], wherein a molar ratio of the metal catalyst to the compound represented by General Formula (II) [metal catalyst / compound represented by General Formula (II)] is 0.01 or more and 0.3 or less.
[20] The production method according to any one of [1] to [19], wherein a molar ratio of the oxidant to the compound represented by General Formula (II) [oxidant / compound represented by General Formula (II)] is 0.5 or more and 20 or less.
[21] The production method according to any one of [1] to [20], wherein a molar ratio of the oxidant to the compound represented by General Formula (II) [oxidant / compound represented by General Formula (II)] is 1 or more and 10 or less.
[22] The production method according to any one of [1] to [21], wherein a molar ratio of the oxidant to the compound represented by General Formula (II) [oxidant / compound represented by General Formula (II)] is 1.2 or more and 5 or less.
[23] The production method according to any one of [1] to [22], wherein the oxidative cleavage is performed at 0 to 100°C.
[24] The production method according to any one of [1] to [23], wherein the compound represented by General Formula (II) is 14-methylbicyclo[10.3.0]pentadecene[1(12)], and the compound represented by General Formula (I) is 3-methyl-1,5-cyclopentadecanedione.
[25] A method for producing a cyclic ketone compound represented by Formula (3), including:
   obtaining 3-methyl-1,5-cyclopentadecanedione using the method according to [22]; and
   Step (a) of obtaining a cyclic ketone compound represented by Formula (3) through partial reduction, followed by dehydration of 3-methyl-1,5-cyclopentadecanedione,
   Step (b) of obtaining a cyclic ketone compound represented by Formula (3) through reduction, followed by enol etherification, and further followed by ring opening of 3-methyl-1,5-cyclopentadecanedione, or
   Step (c) of obtaining a cyclic ketone compound represented by Formula (3) through partial reduction and enol etherification, followed by ring opening of 3-methyl-1,5-cyclopentadecanedione.

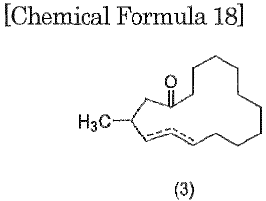

### Yield of Compound

The yield of each compound obtained in the examples was calculated by measuring the mass of the compound in the crude product through gas chromatography internal quantitative analysis and dividing the number of moles of the compound by the number of moles of the raw material.

### Gas Chromatography Equipment and Analytical Conditions

GC system: Model: HP6850 manufactured by Hewlett Packard
Column: DB-1 (inner diameter 0.25 mm, length 30 m, film thickness 0.25 µm) manufactured by J&W
Carrier Gas: He, 1.5 mL/min
Injection conditions: 300°C, split ratio 1/100
Detection conditions: FID method, 300°C
Column temperature conditions: 80°C → increase temperature at 10°C/min → keep at 300°C for 10 min
Internal standard compound: n-tridecane

### Identification of Compound

Each compound obtained in the following examples and comparative examples was identified through spectral analysis using a gas chromatograph mass spectrometer (GC-MS, Model: GC-2010 manufactured by Shimadzu Corporation).

### GC-MS Equipment and Analytical Conditions

GC system: Model: GC-2010 manufactured by Shimadzu Corporation
MS system: Model: GCMS-QP2010 Plus manufactured by Shimadzu Corporation
Column: DB-1 (inner diameter 0.25 mm, length 30 m, film thickness 0.25 µm) manufactured by J&W
Carrier Gas: He, 1.8 mL/min
Injection conditions: 300°C, split ratio 1/50
Detection conditions: FID method, 250°C
Column temperature conditions: 90°C → increase temperature at 5°C/min -> 150°C → increase temperature at 10°C/min → keep at 250°C for 28 min
Ion source temperature: 200°C

### Example 1

A mixture obtained by adding 0.1417 g (2.5 mmol) of 60 mass% hydrogen peroxide solution to 0.0072 g (0.05 mmol) of molybdenum oxide (MoO₃) was stirred at 20°C for 20 minutes. Then, 0.86 g of t-butyl alcohol and 0.22 g (1.0 mmol) of 14-methylbicyclo[10.3.0]pentadecene[1(12)] were added to the mixture and stirred at a reaction temperature of 40°C for 28 hours. After the reaction was completed, 10 ml of 10 mass% aqueous sodium sulfite solution was added to the mixture in an ice bath and quenched. After an organic layer was extracted from the mixture with 30 ml of diethyl ether, the organic layer was dried over magnesium sulfate and filtered, and the solvent was removed from the filtrate under reduced pressure to obtain 0.3 g of crude product. The yield of 3-methyl-1,5-cyclopentadecanedione (compound of Formula (1)) contained in the crude product was 10%. The reaction formula is given below.

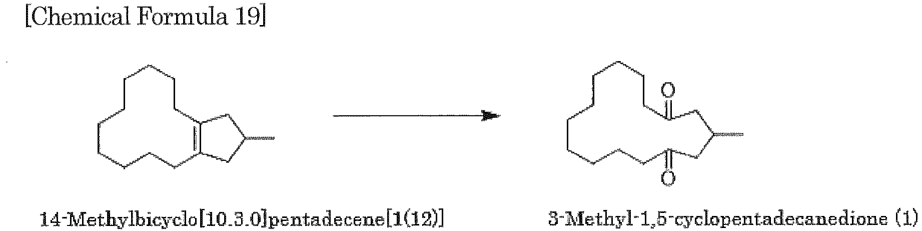

### Examples 2 to 9

The reaction was performed in the same way as that in Example 1, except that the conditions for producing 3-methyl-1,5-cyclopentadecanedione (compound of Formula (1)) were changed as shown in Table 1. Table 1 shows the results.

**Table 1**

| | Oxidant | Metal catalyst | Additive | Reaction time (hours) | Yield of compound of Formula (1) |
|---|---|---|---|---|---|
| Ex. 1 | 60 wt% H₂O₂ (2.5 mmol) | MoO₃ (0.05 mmol) | - | 28 | 10% |
| Ex. 2 | 60 wt% H₂O₂ (2.5 mmol) | H₂MoO₄ (0.05 mmol | - | 24 | 9% |
| Ex. 3 | 60 wt% HzOz (2.5 mmol) | H₂MoO₄ (0.05 mmol) | Oxalic acid (0.2 mmol) | 24 | 19% |
| Ex. 4 | 60 wt% H₂O₂ (2.5 mmol) | MoOs (0.05 mmol) | 3,5-Dichlorosalicylic acid (0.2 mmol) | 30 | 58% |
| Ex. 5 | 60 wt% H₂O₂ (2.5 mmol) | Mo(acac)z (0.05 mmol) | 3,5-Dichlorosalicylic acid (0.2 mmol) | 24 | 45% |
| Ex. 6 | 60 wt% H₂O₂ (2.5 mmol) | H₂MoO₄ (0.05 mmol) | 3,5-Dichlorosalicylic acid (0.2 mmol) | 20 | 41% |
| Ex. 7 | 60 wt% H₂O₂ (2.5 mmol) | VO(acac)₂ (0.05 mmol) | 3,5-Dichlorosalicylic acid (0.2 mmol) | 4 | 6% |
| Ex. 8 | 60 wt% H₂O₂ (2.5 mmol) | Fe(acac)₃ (0.05 mmol) | 3,5-Dichlorosalicylic acid (0.2 mmol) | 4 | 4% |
| Ex. 9 | 70 wt% TBHP (2.5 mmol) | FeCl₃ (0.05 mmol) | - | 22 | 7% |

It was seen from the results in Table 1 above that the method of the present invention can produce a compound of Formula (I) in the presence of a metal catalyst containing one or more metal elements selected from the group consisting of vanadium, iron, and molybdenum.

### Production of Cyclic Ketone Compound Represented by Formula (3)

### Example 10

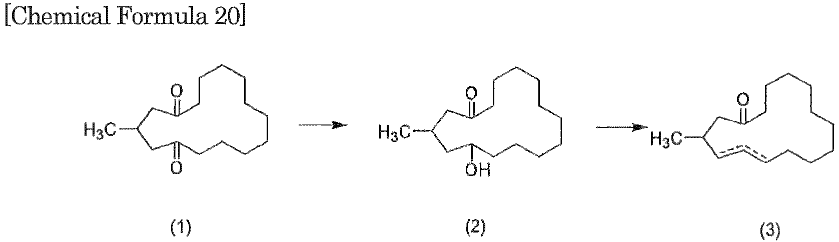

A mixture was obtained by adding 3 ml of methanol, 0.06 g (0.1 mmol) of 10 mass% alcoholic suspension of Raney nickel catalyst, and 0.03 ml of 10 mass% aqueous sodium hydroxide solution to 0.15 g (0.594 mmol) of 3-methyl-1,5-cyclopentadecanedione (compound of Formula (1)) obtained in Example 1. The mixture was then stirred under hydrogen at room temperature (25°C) and normal pressure (0.1 MPa) for 45 minutes and filtered, and the solvent was removed from the obtained filtrate under reduced pressure. The residue was diluted with diethyl ether and washed with 10 mass% aqueous sodium hydrogen carbonate solution and water. After an organic layer was extracted, the organic layer was dried over magnesium sulfate and filtered, and the solvent was removed from the filtrate under reduced pressure to obtain 0.2 g of crude product. It was found from an analysis on the crude product by gas chromatography that the yield of the obtained 3-methylcyclopentadecanol-5-one (compound of Formula (2)) was 50.0%.

A mixture obtained by adding 3 ml of toluene and 0.004 g (0.027 mmol) of benzenesulfonic acid to 0.07 g (0.275 mmol) of 3-methylcyclopentadecanol-5-one (compound of Formula (2)) was stirred for 1 hour under heat reflux (110°C). Then, 10 mass% aqueous sodium hydrogen carbonate solution was added at room temperature (25°C). After an organic layer was extracted, the organic layer was dried over magnesium sulfate and filtered, and the solvent was removed from the filtrate under reduced pressure to obtain 0.06 g of crude product. It was found from an analysis on the crude product by gas chromatography that the yield of the obtained cyclic ketone compound represented by Formula (3) was 80.0%.

### Example 11

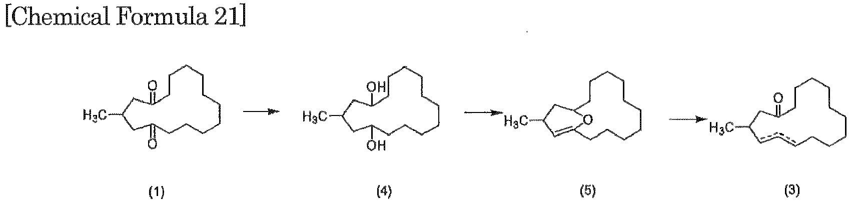

A mixture was obtained by adding 3 ml of methanol, 0.06 g (0.1 mmol) of 10 mass% alcoholic suspension of Raney nickel catalyst, and 0.03 ml of 10 mass% aqueous sodium hydroxide solution to 0.15 g (0.594 mmol) of 3-methyl-1,5-cyclopentadecanedione (compound of Formula (1)) obtained in Example 1. The mixture was then stirred under hydrogen at room temperature (25°C) and normal pressure (0.1 MPa) for 24 hours and filtered, and the solvent was removed from the obtained filtrate under reduced pressure. The residue was diluted with diethyl ether and washed with 10 mass% aqueous sodium hydrogen carbonate solution and water. After an organic layer was extracted, the organic layer was dried over magnesium sulfate and filtered, and the solvent was removed from the filtrate under reduced pressure to obtain 0.2 g of crude product. It was found from an analysis on the crude product by gas chromatography that the yield of the obtained 3-methylcyclopentadecane-1,5-diol (compound of Formula (4)) was 80.0%.

A mixture obtained by adding 0.06 g (0.09 mmol) of 10 mass% aqueous suspension of Raney copper to 0.12 g (0.46 mmol) of 3-methylcyclopentadecane-1,5-diol (compound of Formula (4)) was stirred at 165°C for 3 hours under a pressure reduced to 45 mmHg. Then, 0.06 g of distillation fraction was obtained through distillation under a pressure reduced to 2 mmHg. It was found from an analysis on the distillation fraction by gas chromatography that the yield of the obtained 16-oxa-3-methylbicyclo[10.3.1]pentadec-1-ene (compound of Formula (5)) was 50.0%.

A mixture obtained by adding 1 ml of toluene and 0.01 g (0.08 mmol) of 80 mass% aqueous phosphoric acid solution to 0.05 g (0.21 mmol) of 16-oxa-3-methylbicyclo[10.3.1]pentadec-1-ene (compound of Formula (5)) was stirred for 3 hours under heat reflux (110°C) while continuously removing by-product water. After cooling, the mixture was washed with water and 10 mass% aqueous sodium carbonate solution, an organic layer was extracted, and the solvent was removed under reduced pressure to obtain 0.05 g of crude product. It was found from an analysis on the crude product by gas chromatography that the yield of the obtained cyclic ketone compound represented by Formula (3) was 85.0%.

The present invention can provide a production method for producing a compound represented by General Formula (I) through oxidative cleavage of a compound of Formula (II), which is a bicyclic tetrasubstituted olefin compound.

## Claims

1. A method for producing a compound represented by General Formula (I), comprising a step of obtaining a compound represented by General Formula (I) through oxidative cleavage of a compound represented by General Formula (II) using an oxidant in the presence of a metal catalyst containing one or more metal elements selected from the group consisting of vanadium, iron, and molybdenum: 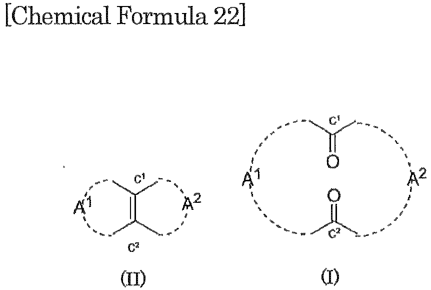 wherein, in Formulas (I) and (II) above,
Formula -A¹- (wherein a former bonding hand means a bonding hand that is bonded to a carbon atom C¹, and a latter bonding hand means a bonding hand that is bonded to a carbon atom C²) is an alkylene group with 2 or more and 6 or less of carbon atoms that is optionally substituted and that optionally further contains an ether bond, an ester bond, a secondary amino group, a thioether group, or a combination thereof, and
Formula -A²- (wherein a former bonding hand means a bonding hand that is bonded to a carbon atom C¹, and a latter bonding hand means a bonding hand that is bonded to a carbon atom C²) is an alkylene group with 4 or more and 10 or less of carbon atoms that is optionally substituted and that optionally further contains an ether bond, an ester bond, a secondary amino group, a thioether group, or a combination thereof.

2. The production method according to claim 1,
wherein Formula -A¹- is an alkylene group with 3 or 4 carbon atoms that is optionally substituted, and
Formula -A²- is an alkylene group with 4, 6, 8 or 10 carbon atoms that is optionally substituted.

3. The production method according to claim 1 or 2, wherein the metal catalyst is one or more selected from a molybdic acid compound and a molybdenum complex compound.

4. The production method according to any one of claims 1 to 3, wherein the metal catalyst is one or more selected from a vanadic acid compound, a vanadate, a vanadium alkoxide compound, and a vanadium complex compound.

5. The production method according to any one of claims 1 to 4, wherein the metal catalyst is one or more selected from iron oxide, iron nitrate, iron sulfate, iron alkoxide, iron carboxylate, iron halide, and iron complex.

6. The production method according to any one of claims 1 to 5, wherein the metal catalyst is selected from the group consisting of a vanadium(IV) compound, a vanadium(V) compound, a molybdenum(VI) compound, an iron(II) compound, and an iron(III) compound.

7. The production method according to any one of claims 1 to 6, wherein the metal catalyst is selected from the group consisting of molybdenum(VI) oxide, molybdic acid, dioxomolybdenum(VI) complex, polymolybdic acid, and heteropolymolybdic acid.

8. The production method according to any one of claims 1 to 7, wherein the oxidant contains hydrogen peroxide or tert-butyl hydroperoxide.

9. The production method according to any one of claims 1 to 8, wherein a molar ratio of the metal catalyst to the compound represented by General Formula (II) [metal catalyst / compound represented by General Formula (II)] is 0.0001 or more and 1 or less.

10. The production method according to any one of claims 1 to 9, wherein a molar ratio of the oxidant to the compound represented by General Formula (II) [oxidant / compound represented by General Formula (II)] is 0.5 or more and 20 or less.

11. The production method according to any one of claims 1 to 10, wherein the oxidative cleavage is performed at 0 to 100°C.

12. The production method according to any one of claims 1 to 11, wherein the oxidative cleavage uses a compound represented by Formula (III) below as an additive: 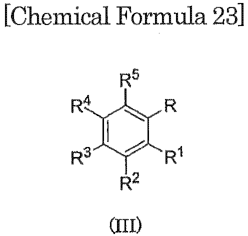 wherein, in Formula (III) above,
R is COOH or OH, and
R¹, R², R³, R⁴, and R⁵ are each independently selected from the group consisting of hydrogen, a halogen atom, NO₂, OH, COOH, NR⁶R⁷ (wherein R⁶ and R⁷ are each independently hydrogen, an alkyl group with 1 or more and 3 or less of carbon atoms, or an alkyl group with 1 or more and 3 or less of carbon atoms substituted with OH), an alkyl group with 1 or more and 6 or less of carbon atoms, an alkoxy group with 1 or more and 6 or less of carbon atoms, and a phenyl group (which is optionally substituted), or
two of R¹, R², R³, R⁴, and R⁵, taken together with the carbon atoms carrying them, form a saturated or unsaturated hydrocarbon ring, and the other three are each independently selected from the group consisting of hydrogen, a halogen atom, NO₂, OH, COOH, NR⁶R⁷ (wherein R⁶ and R⁷ are each independently hydrogen, an alkyl group with 1 or more and 3 or less of carbon atoms, or an alkyl group with 1 or more and 3 or less of carbon atoms substituted with OH), an alkyl group with 1 or more and 6 or less of carbon atoms, an alkoxy group with 1 or more and 6 or less of carbon atoms, and a phenyl group (which is optionally substituted).

13. The production method according to claim 12, wherein the additive contains one or more selected from benzenecarboxylic acid, benzenedicarboxylic acid, and benzenehydroxycarboxylic acid that are optionally substituted with a halogen atom, NO₂, or an alkoxy group with 1 or more and 6 or less of carbon atoms.

14. The production method according to claim 12 or 13, wherein the additive contains salicylic acid that is optionally substituted with a halogen atom, NO₂, or an alkoxy group with 1 or more and 6 or less of carbon atoms.

15. The production method according to any one of claims 1 to 14, wherein the compound represented by General Formula (II) is 14-methylbicyclo[10.3.0]pentadecene[1(12)], and the compound represented by General Formula (I) is 3-methyl-1,5-cyclopentadecanedione.

16. A method for producing a cyclic ketone compound represented by Formula (3), comprising
obtaining 3-methyl-1,5-cyclopentadecanedione using the method according to claim 15; and
Step (a) of obtaining a cyclic ketone compound represented by Formula (3) through partial reduction, followed by dehydration of 3-methyl-1,5-cyclopentadecanedione,
Step (b) of obtaining a cyclic ketone compound represented by Formula (3) through reduction, followed by enol etherification, and further followed by ring opening of 3-methyl-1,5-cyclopentadecanedione, or
Step (c) of obtaining a cyclic ketone compound represented by Formula (3) through partial reduction and enol etherification, followed by ring opening of 3-methyl-1,5 -cyclopentadecanedione:
